# EUROPÄISCHE PATENTSCHRIFT

(11) **EP 1 844 800 B1**
(45) Veröffentlichungstag und Bekanntmachung des Hinweises auf die Patenterteilung: **25.02.2009**
(21) Anmeldenummer: 06112431.9
(22) Anmeldetag: 10.04.2006
(51) Int. Cl.: A61M 1/34, A61M 1/16, A61M 1/36, A61B 5/02

(54) **Therapieeinrichtung mit zeitflexibler Blutdruckregelung**
Therapeutic device with control of the blood pressure at arbitrary points in time
Dispositif thérapeutique avec contrôle de la pression artérielle à des moments arbitraires

(43) Veröffentlichungstag der Anmeldung: 17.10.2007
(73) Patentinhaber: B. Braun Avitum AG, 49215 Glandorf (DE)
(72) Erfinder: Röher, Otfried, 01139 Dresden (DE); Korth, Steffen, 99334 Elleben (DE)
(74) Vertreter: Selting, Günther

(56) Entgegenhaltungen:
- EP-A- 0 956 872
- EP-A- 1 226 838
- US-A- 4 718 891
- US-B1- 6 736 789

## Beschreibung

Die Erfindung betrifft eine Therapieeinrichtung mit einem Blutdruckmessgerät und einer Regeleinrichtung mit zeitflexibler Regelung des Blutdruckes des Patienten während eines Therapievorganges, insbesondere ein Therapiegerät zur extrakorporalen Blutreinigung.

Bei verschiedenen Therapien, wie beispielsweise bei der Hämodialyse, ist es erforderlich, den Blutdruck des Patienten laufend zu überwachen. Hierfür werden Geräte zur unblutigen (non-invasiven) Blutdruckmessung mit einer um den Arm des Patienten herumlegbaren Manschette benutzt, die aufgepumpt und dann unter Druckmessung langsam entlastet wird. Um eine quasi-kontinuierliche Blutdruckregelung zu erreichen, muss die Regeleinrichtung in Intervallen von wenigen Minuten Regelvorgänge durchführen. Die während einer mehrstündigen Behandlung hierfür erforderliche Anzahl von Blutdruckmessungen kann erheblich reduziert werden, wenn zu festgelegten Zeitpunkten die Blutdruckmessungen durch Blutdruckwerte substituiert werden, die aus früheren Behandlungen desselben Patienten gewonnen wurden.

In der Europäischen Patentanmeldung EP 1 226 838 A2 sind Therapieeinrichtungen mit intervallartiger Blutdruckmessung und mit reduzierter Anzahl der Messvorgänge beschrieben. Anhand der gleichen Länge aller Intervalle, beispielsweise 5 Minuten je Intervall, gewährleistet hierbei die Regeleinrichtung die Auswertung des Blutdruckverlaufes und der Blutdrucktrends in jedem Intervall nach einheitlichen Regeln, wie dies in EP 0 956 872 B1 beschrieben ist.

Da für morbide Ereignisse während der Hämodialyse, wie Hypotonie, Schwindelanfälle, Erbrechen u. a., multifaktorielle Ursachen zu berücksichtigen sind, werden in der klinischen Praxis mitunter Monitore für weitere Größen (beispielsweise EKG, relatives Blutvolumen, Hämatokrit, Blutsauerstoffgehalt) zur Erkennung von Komplikationen verwendet. Hieraus können sich in Abhängigkeit von den Messergebnissen zu beliebigen Zeitpunkten Anforderungen für zusätzliche Blutdruckmessungen ergeben. Klinische Erfahrungen mit Dialysepatienten zeigen, dass in Abhängigkeit vom Zustand des Patienten mitunter auch Blutdruckmessungen direkt vom medizinischen Personal angefordert werden.

Der Erfindung liegt deshalb die Aufgabe zugrunde, eine Therapieeinrichtung mit reduzierter Anzahl der Blutdruckmessungen derart auszubilden, dass eine zeitflexible Blutdruckregelung ermöglicht wird.

Die Lösung dieser Aufgabe erfolgt erfindungsgemäß mit den Merkmalen des Patentanspruchs 1. Hiernach wird von der Regeleinrichtung nach jeder Blutdruckmessung in Abhängigkeit vom ermittelten Blutdruckwert entschieden, ob die Blutdruckregelung auf der Grundlage von hypothetischen oder von realen Blutdruckwerten fortgesetzt wird. Dazu werden von der Regeleinrichtung die folgenden Kategorien von Blutdruckwerten in die Regelung des Blutdruckes einbezogen:
a) Blutdruckwerte, die von der Regeleinrichtung entsprechend einem implementierten Zeitregime in Abhängigkeit von den an der Therapieeinrichtung eingestellten Zielparametern der Behandlung (zum Beispiel Gesamt-Ultrafiltrationsvolumen, Behandlungsdauer, maximale Ultrafiltrationsrate) zu vorgesehenen Zeitpunkten durch reguläre Trigger (RT) angefordert oder durch Werte aus früheren Therapievorgängen substituiert werden,
   dadurch gekennzeichnet, dass die Regeleinrichtung auch die folgenden Kategorien von Blutdruckwerten in die Regelung des Blutdrucks einbezieht:
b) Blutdruckwerte, die von angeschlossenen externen Mess- und Auswertegeräten für andere physiologische Größen (zum Beispiel EKG, Relatives Blutvolumen, Hämatokrit, Blutsauerstoffgehalt) zu anderen Zeitpunkten als unter a) durch irreguläre Trigger (IRT) und/oder durch quasi-reguläre Trigger (QRT) automatisch angefordert werden, wobei irreguläre Trigger solche sind, die in einem größeren Zeitabstand vom Zeitpunkt eines regulären Triggers auftreten und quasi-reguläre Trigger solche sind, die in einem kleineren Zeitabstand vom Zeitpunkt eines regulären Triggers auftreten, und
c) Blutdruckwerte, die vom medizinischen Personal manuell zu anderen Zeitpunkten als unter a) durch irreguläre Trigger (IRT) und/oder durch quasi-reguläre Trigger (QRT) angefordert werden.

Vorzugsweise führt die Regeleinrichtung die folgenden Schritte aus:
- A: Erfassung, Klassierung und Verarbeitung aller nach den Kategorien a) bis c) ermittelten Blutdruckmesswerte in Abhängigkeit von den jeweiligen Zeitpunkten der Messungen und entsprechend dem von der Regeleinrichtung vorgegebenen Zeitregime für die Blutdruckregelung,
- B: Ersetzen aller Blutdruckwerte nach Kategorie a) durch die ermittelten Blutdruckmesswerte nach Kategorien b) und c), wenn diese Messwerte innerhalb eines festgelegten Toleranzzeitraumes unmittelbar vor den für die Blutdruckwerte nach Kategorie a) festgelegten Zeitpunkten vom Blutdruckmesser an die Regeleinrichtung übergeben werden.
- C: Erstellen und Speichern einer Sammlung der zeitlichen Verläufe des Blutdruckes bei mehreren Therapievorgängen,
- D: Ermitteln desjenigen Blutdruckverlaufes aus den gespeicherten Verläufen, der mit dem aktuellen Blutdruckverlauf die größte Ähnlichkeit hat, als Leitkurve,
- E: Regelung des Blutdruckes anhand der nach den Kategorien a) bis c) erhaltenen Blutdruckwerte, wobei die Werte für vorgesehene Zeitpunkte ohne Blutdruckmessung aus der Leitkurve ermittelt werden.

Gemäß einer bevorzugten Weiterbildung der Erfindung ist vorgesehen, dass die von extern angeschlossenen Geräten oder vom medizinischen Personal angeforderten Blutdruckmessungen in Abhängigkeit von ihrem Zeitpunkt innerhalb der von der Regeleinrichtung festgelegten Regelintervalle unterschiedlich bewertet werden. Eine extern angeforderte Blutdruckmessung in geringem Zeitabstand, beispielsweise 60 sec, vor Beginn eines neuen Regelintervalls wird einer regulären Blutdruckmessung gleichgestellt, wie sie ohne externe Anforderung von der Regeleinrichtung selbst zu den hierfür festgelegten Zeitpunkten durch einen regulären Trigger (RT) veranlasst würde. Der extern ausgelöste Trigger wird deshalb als quasi-regulärer Trigger (QRT) gewertet. Andere extern angeforderte Blutdruckmessungen in größerem Zeitabstand, beispielsweise > 60 sec vor Beginn eines neuen Regelintervalls, werden von der Regeleinrichtung als irreguläre Trigger (IRT) erkannt und durch eine separate Regelbasis bewertet, die die Zeitpunkte innerhalb des jeweiligen Regelintervalls berücksichtigt. Bei mehreren externen Anforderungen innerhalb eines Regelintervalls reagiert diese Regelbasis auf jede einzelne Anforderung sofort mit einer Anpassung der Führungsgröße der Blutdruckregelung, beispielsweise der Ultrafiltrationsrate. Als Bezugswert hierfür dient unabhängig von der Anzahl externer Anforderungen und in gleicher Weise wie für reguläre Blutdruckwerte jeweils der Wert der Führungsgröße, den die Regeleinrichtung zu Beginn des betreffenden Regelintervalls ermittelt hat. Sobald eine extern angeforderte Blutdruckmessung zu einem Blutdruckwert im regelpflichtigen Blutdruckbereich führt, veranlasst die Regeleinrichtung zu Beginn des nächsten Regelintervalls selbst eine weitere Blutdruckmessung. Diese Blutdruckmessung wird auch ohne externe Anforderung in den darauf folgenden Regelintervallen in gleicher Weise wie nach regulären Messungen solange wiederholt, bis der Blutdruck wieder oberhalb des regelpflichtigen Blutdruckbereiches liegt. Durch die beschriebene Arbeitsweise gewährleistet die Regeleinrichtung eine zeitflexible Blutdruckregelung, die unabhängig vom Zeitpunkt und von der Anzahl externer Anforderungen sofort nach jeder Blutdruckmessung reagiert und damit auch in kritischen Situationen die Qualität der Blutdruckregelung und die Sicherheit des Patienten erhöht. Gleichzeitig werden unnötige Doppelmessungen am Patienten vermieden, wenn externe und reguläre Anforderungen für Blutdruckmessungen in einem geringen Zeitabstand aufeinander folgen.

Die Erfindung eignet sich insbesondere für Dialysegeräte und andere Geräte für die extrakorporale Blutbehandlung, jedoch auch beispielsweise für die Infusionstherapie.

Im folgenden wird unter Bezugnahme auf die Zeichnungen ein Ausführungsbeispiel der Erfindung näher erläutert.

Es zeigen:
- Fig. 1: eine schematische Darstellung einer Therapieeinrichtung in Form eines Dialysegerätes,
- Fig. 2: ein Zeitdiagramm der Dialysebehandlung mit Darstellung des Blutdruckes und der Ultrafiltrationsrate,
- Fig. 3: ein Zeitdiagramm mit regulärer Reaktion der Regeleinrichtung ohne externe Messanforderungen und
- Fig. 4: ein Zeitdiagramm mit zeitflexibler Reaktion der Regeleinrichtung bei externen Messanforderungen.

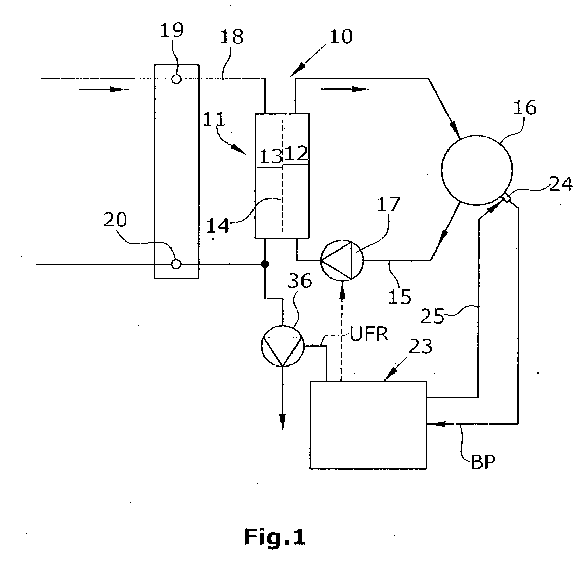

Das in Fig. 1 dargestellte Dialysegerät entspricht in seinem Grundaufbau demjenigen von EP 0 956 872 B1 oder von EP 1 226 838 A2. Das Dialysegerät 10 enthält eine Ultrafiltrationsvorrichtung 11 mit einer Primärkammer 12 und einer Sekundärkammer 13, die durch eine Membran 14 voneinander getrennt sind. Die Primärkammer 12 ist Bestandteil eines Blutkreislaufes 15, bei dem Blut, welches über das arterielle System des Patienten entnommen wurde, in der Ultrafiltrationsvorrichtung 11 gereinigt und anschließend über das venöse System zum Patienten 16 zurückgeführt wird. In dem Blutkreislauf 15 befindet sich eine Pumpe 17. Diese ist als volumetrische Pumpe ausgebildet, d. h. die Fördermenge dieser Pumpe entspricht ihrer Antriebsgeschwindigkeit, und ist steuerbar.

Die Sekundärkammer 13 der Ultrafiltrationsvorrichtung 11 befindet sich in einem Dialysierflüssigkeitsweg 18, in dem Dialysierflüssigkeit gepumpt wird. Die Dialysierflüssigkeit kommt von einem (nicht dargestellten) Vorratsbehälter, nimmt in der Ultrafiltrationsvorrichtung 11 zusätzliche Substanzen aus dem Blut auf und wird dann zu einem (nicht dargestellten) Ablauf gepumpt. In dem Dialysierflüssigkeitsweg ist vor und hinter der Sekundärkammer 13 jeweils eine Durchflusskammer 19 bzw. 20 angeordnet, welche die Durchflussrate an der betreffenden Stelle regelt. Die Durchflusskammer 19 und die Durchflusskammer 20 haben die gleiche Förderrate. Über die volumengesteuerte Ultrafiltrationspumpe 36 wird die gewünschte Ultrafiltrationsmenge UFV in einer festgelegten Ultrafiltrationsrate UFR abgezogen. Das Zeitintegral über die Ultrafiltrationsrate UFR bildet das Ultrafiltrationsvolumen UFV, also dasjenige Flüssigkeitsvolumen, das seit Beginn der Behandlung die Membran 14 passiert hat. Die Regelung der Ultrafltrationsrate erfolgt durch die Regeleinrichtung 23, die Steuersignale für die Förderrate der Pumpe 36 liefert. Die Förderrate der Pumpe wird in der Weise eingestellt, dass sich eine gewünschte Ultrafiltrationsrate ergibt.

Die Regeleinrichtung 23 empfängt ferner das Blutdrucksignal BP eines Blutdruckmessers 24, der am Körper des Patienten angebracht ist. Der Blutdruckmesser weist eine aufblasbare Manschette auf, die um den Arm des Patienten gelegt ist, und führt nicht-invasive Blutdruckmessungen aus. Die Zeitpunkte der Blutdruckmessungen werden intern durch die Regeleinrichtung 23 über die Leitungen 25 und extern durch angeschlossene Monitore und/oder das medizinische Personal festgelegt. Neben dem Blutdruckwert können der Regeleinrichtung 23 auch noch Blutdrucktrendwerte zugeführt werden, wie es in EP 0 956 872 B1 beschrieben ist. Die Regeleinrichtung 23 regelt in Abhängigkeit von den erhaltenen Eingangsgrößen die lJltrafiltrationsrate UFR.

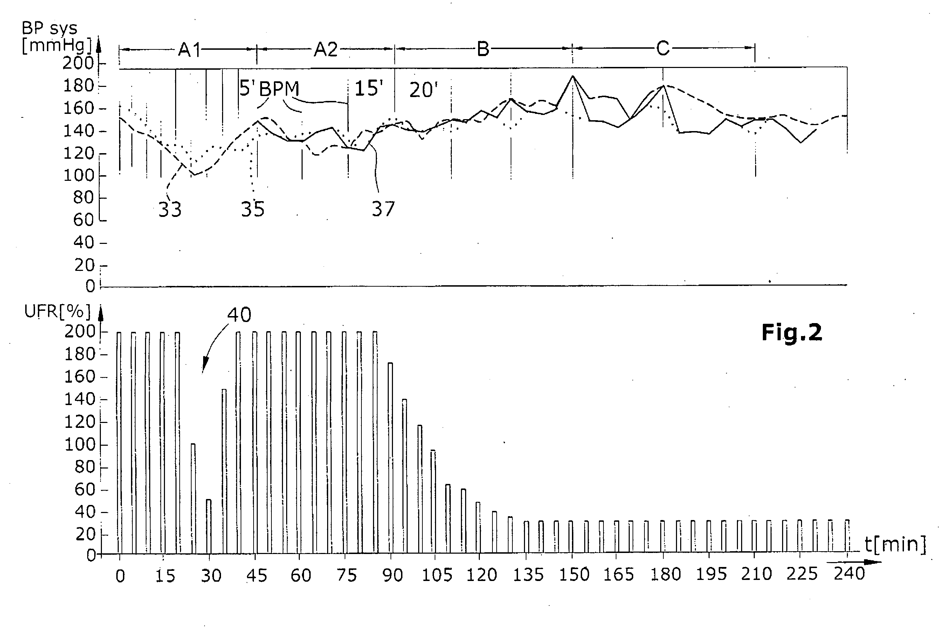

Fig 2 veranschaulicht ein Beispiel der Blutdrockregelung, wie sie von der Regeleinrichtung 23 ohne externe Anforderungen für zusätzliche Blutdruckmessungen während eines Therapievorganges gesteuert wird.

Die untere Hälfte von Fig. 2 zeigt den zeitlichen Verlauf der relativen Ultrafiltrationsrate (in %). Während dieser Dialysebehandlung wird zunächst mit hoher relativer Ultrafiltrationsrate von 200 % gearbeitet, und anschließend wird die Ultrafiltrationsrate auf einen Minimalwert von etwa 25 % abgesenkt.

In der oberen Hälfte von Fig. 2 ist der systolische Blutdruck BPsys eines Patienten während dieser Dialysebehandlung eingetragen. Dabei zeigt die gestrichelte Linie den aktuellen Blutdruckverlauf 33, wie er sich bei kontinuierlicher Blutdruckmessung ergeben würde.

In der Regeleinrichtung 23 oder in einem zentralen Computer ist eine Sammlung von zeitlichen Verläufen des Blutdruckes bei früheren Therapievorgängen gespeichert.

In einer Anfangsphase A1 wird mit dem Blutdruckmessgerät 24 in Intervallen von 5 min der aktuelle Blutdruck gemessen und als Blutdruckverlauf 33 gespeichert. Die Blutdruckregelung erfolgt ausschließlich in Abhängigkeit von dem aktuellen Blutdruckverlauf 33. Die Anfangsphase dauert bei dem vorliegenden Ausführungsbeispiel 45 min. Am Ende der Anfangsphase A1 wird der aktuelle Blutdruckverlauf 33 mit den gespeicherten Blutdruckverläufen vorangegangener Behandlungen desselben Patienten verglichen. Anhand statistischer Analysen (Mittelwerte, Standardabweichungen, t-Test oder Bildverarbeitungsmethoden) wird aus dem Datenspeicher der Blutdruckverlauf mit der größten Ähnlichkeit zum aktuellen Blutdrockverlauf ermittelt und für die laufende Behandlung als (gepunktete) Leitkurve 35 benutzt.

In der sich anschließenden zweiten Phase A2 werden Blutdruckmessungen, die durch die Striche BPM bezeichnet sind, im Zeitregime der Regeleinrichtung 23 durchgeführt, im vorliegenden Fall in Intervallen von 15 min. In der Phase B erfolgen die Blutdruckmessungen BPM in Intervallen von 20 min oder in der Endphase C in Intervallen von 30 min. Die Blutdruckregelung findet jedoch weiterhin in Intervallen von 5 min statt, wobei jeweils zu den Zeitpunkten, in denen keine Messung durchgeführt wird, die Werte der (gepunkteten) Leitkurve als Ist-Wert des Blutdruckes zu Grunde gelegt werden und in den Zeitpunkten der Blutdruckmessung BPM die aktuellen Werte des (gestrichelten) Blutdruckverlaufes 33. Die als Ist-Blutdruck für die Regelung benutzten Werte ergeben sich aus der durchgezogenen Kurve 37, die den "hypothetischen Blutdruckverlauf" angibt.

Aus Fig. 2 ist zu erkennen, dass zu den Zeitpunkten der Blutdruckmessung BPM der hypothetische Blutdruckverlauf, der für die Regelung maßgebend ist, den Wert des aktuellen (gestrichelten) Blutdruckes 33 annimmt, während in den dazwischen liegenden Zeitpunkten der Wert der (gepunkteten) Leitkurve angenommen wird.

Wenn der Ist-Wert unter einen vorbestimmten Grenzwert, in diesem Beispiel 130 mmHg, abfällt, wird die Ultrafiltrationsrate UFR verringert, so wie das in Fig. 2 in dem Bereich 40 angegeben ist. Durch die Verringerung der Ultrafiltrationsrate wird dem Patienten weniger Flüssigkeit entzogen, und auf diese Weise wird der Verringerung des Blutdruckes entgegengewirkt.

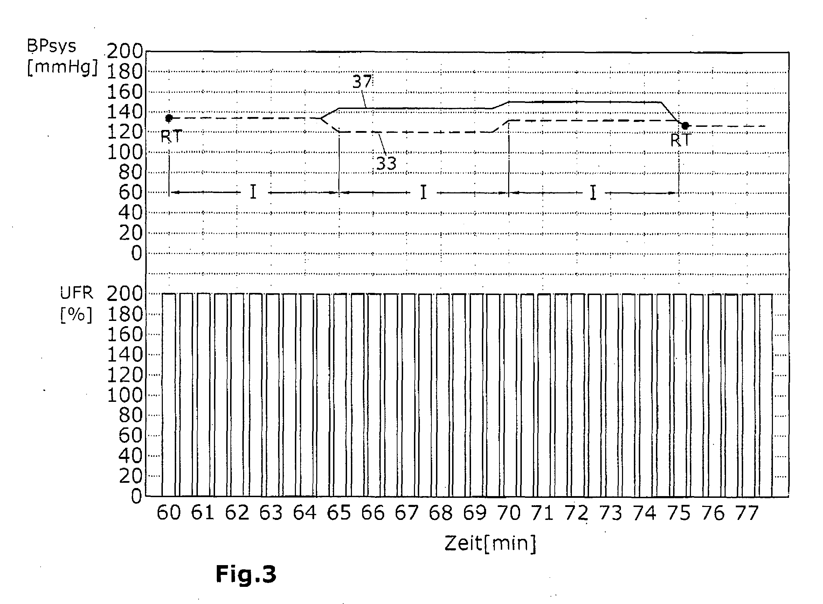

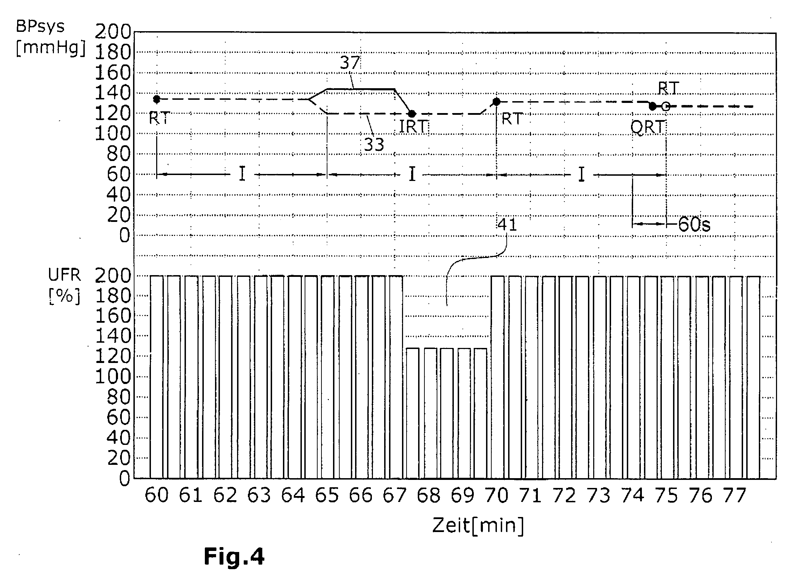

Fig. 3 und Fig. 4 veranschaulichen für den in Fig. 2 enthaltenen Behandlungsabschnitt von 60 min bis 77 min über einer gespreizten Zeitachse die zeitflexible Reaktion der Regeleinrichtung 23 auf extern angeforderte Blutdruckmessungen. Die Regeleinrichtung bewirkt eine Aufteilung der Zeitachse in Intervalle I, die hier 5 min betragen und jeweils von 60 - 65 min, von 65 - 70 min und von 70 - 75 min usw. dauern. Ohne externe Anforderungen (Fig. 3) veranlasst die Regeleinrichtung 23 in Behandlungsphase A2 nur im Abstand von 15 min, also nach jeweils 3 Intervallen, Messungen durch die regulären Trigger RT zu den Zeitpunkten 60 min und 75 min gemäß Kriterium a). In dem auf einen regulären Trigger RT folgenden ersten Intervall von 60 - 65 min erfolgt die Blutdruckregelung anhand der gemessenen Werte 33. In dem zweiten und dritten Intervall I von 60 min bis 70 min und von 70 min bis 75 min erfolgt die Blutdruckregelung mit den hypothetischen Werten 37.

Der gemessene Blutdruckwert 33 wird während des ersten Intervalls in jeder Zeitspanne für die Regelung zugrunde gelegt. In den nachfolgenden Intervallen derselben Zeitspanne erfolgt die Regelung anhand der Kurve 37, die den "hypothetischen Blutdruckverlauf" angibt. Auf diese Weise werden häufige Blutdruckmessungen vermieden. Wenn allerdings gemäß Figur 4 während der Benutzung der Kurve 37 ein irregulärer Trigger IRT auftritt, kann die Regelung zu der aktuellen Kurve 33 zurückkehren. Somit wird mit dem irregulären Trigger IRT eine neue Blutdruckmessung ausgelöst, was dann zur Folge hat, dass zur Zeit 70 min, also am Anfang des dritten Intervalls I, wiederum eine Blutdruckmessung durchgeführt wird.

Die zeitflexible Reaktion der Regeleinrichtung in Abhängigkeit von den Zeitpunkten externer Anforderungen veranschaulicht Figur 4. Die eingetragene externe Anforderung gemäß Kategorie b) oder c) zum Zeitpunkt 67,5 min löst einen irregulären Trigger IRT aus, da der Anforderungszeitpunkt außerhalb des Toleranzzeitraums von 60 sec vor dem Beginn des nächsten 5-min-Intervalls bei 70 min liegt. Die Regeleinrichtung reagiert sofort mit einer Senkung der Ultrafiltrationsrate UFR im Zeitabschnitt 41, da der gemessene Blutdruck < 130 mmHg beträgt und im regelpflichtigen Blutdruckbereich liegt. Bei einer Absenkung der Ultrafiltrationsrate werden die regulären Trigger RT in kürzeren Zeitabständen erzeugt, hier in den Zeitabständen eines Intervalls I. Die Regeleinrichtung 23 veranlasst also bereits zum Beginn des nächsten 5-min-Intervalls I bei 70 min einen regulären Trigger RT und damit eine weitere reguläre Blutdruckmessung. Da der Messwert hier > 130 mmHg ist, erhöht die Regeleinrichtung 23 jetzt die relative Ultrafiltrationsrate UFR wieder auf 200 %. Die eingetragene externe Messanforderung bei 74,5 min erzeugt einen quasiregulären Trigger QRT, da der Anforderungszeitpunkt hier im Toleranzzeitraum von 60 s vor dem Beginn des nächsten 5-min-Intervalls I liegt. Der sonst fällige reguläre Trigger bei 75 min wird deshalb unterdrückt und der vom quasiregulären Trigger QRT ermittelte Blutdruckwert = 130 mmHg für das nächste 5-min-Intervall von 75 min bis 80 min übernommen. Die hypothetischen Werte 37 werden in diesem Beispiel nur im Zeitabschnitt von 65,0 min bis 67,5 min wirksam. In den Zeitabschnitten von 60,0 min bis 65,0 min und von 67,5 min bis 80,0 min erfolgt die Regelung anhand des aktuellen Blutdruckverlaufs 33.

## Patentansprüche

1. Therapieeinrichtung für eine Behandlung mit einstellbaren Zielparametern, wie Gesamt-Ultrafiltrationsvolumen, Behandlungsdauer, maximale Ultrafiltrationsrate, mit einem Blutdruckmessgerät (24) und einer Regeleinrichtung (23) zur zeitflexiblen Regelung des Blutdruckes des Patienten während eines Therapievorganges in Abhängigkeit von ermittelten Blutdruckwerten, wobei die Regeleinrichtung nach jeder Blutdruckmessung in Abhängigkeit vom ermittelten. Blutdruckwert entscheidet, ob die Blutdruckregelung mit hypothetischen oder mit realen Blutdruckwerten fortgesetzt wird, und wobei die Regeleinrichtung (23) die folgende Kategorie von Blutdruckwerten in die Regelung des Blutdruckes einbezieht:
a) Blutdruckwerte, die von der Regeleinrichtung entsprechend einem implementierten Zeitregime in Abhängigkeit von den an der Therapieeinrichtung eingestellten Zielparametern der Behandlung zu vorgesehenen Zeitpunkten durch reguläre Trigger (RT) angefordert oder durch Werte aus früheren Therapievorgängen substituiert werden,
**dadurch gekennzeichnet, dass**
die Regeleinrichtung (23) auch die folgenden Kategorien von Blutdruckwerten in die Regelung des Blutdrucks einbezieht:
b) Blutdruckwerte, die von Mess- und Auswertegeräten für andere physiologische Größen zu anderen Zeitpunkten als unter a) durch irreguläre Trigger (IRT) und/oder quasi-reguläre Trigger (QRT) angefordert werden, wobei irreguläre Trigger solche sind, die in größerem Zeitabstand vom Zeitpunkt des nächsten regulären Triggers auftreten und quasi-reguläre Trigger solche sind die in einem kleineren Zeitabstand vom Zeitpunkt des nächsten regulären Triggers auftreten, und
c) Blutdruckwerte, die vom medizinischen Personal zu anderen Zeitpunkten als unter a) durch irreguläre Trigger (IRT) und/oder quasi-reguläre Trigger (QRT) angefordert werden.

2. Therapieeinrichtung nach Anspruch 1, wobei die Regeleinrichtung (23) die folgenden Schritte ausführt:
A Erfassung, Klassierung und Verarbeitung aller nach den Kategorien a) bis c) ermittelten Blutdruckmesswerte in Abhängigkeit von den jeweiligen Zeitpunkten der Messungen und entsprechend dem von der Regeleinrichtung (23) vorgegebenen Zeitregime für die Blutdruckregelung,
B Ersetzen aller Blutdruckwerte nach Kategorie a) durch die ermittelten Blutdruckmesswerte nach Kategorien b) und c), wenn diese Messwerte innerhalb eines festgelegten Toleranzzeitraumes unmittelbar vor den für die Blutdruckwerte nach Kategorie a) festgelegten Zeitpunkten vom Blutdruckmesser an die Regeleinrichtung übergeben werden.
C Erstellen und Speichern einer Sammlung der zeitlichen Verläufe des Blutdruckes bei mehreren Therapievorgängen,
D Ermitteln desjenigen Blutdruckverlaufes aus den gespeicherten Verläufen, der mit dem aktuellen Blutdruckverlauf (33) die größte Ähnlichkeit hat, als Leitkurve (35),
E Regelung des Blutdruckes anhand der nach den Kategorien a) bis c) erhaltenen Blutdruckwerte, wobei die Werte für vorgesehene Zeitpunkte ohne Blutdruckmessung aus der Leitkurve (35) ermittelt werden.

3. Therapieeinrichtung nach Anspruch 2, wobei die Regeleinrichtung nach jeder Blutdruckmessung eine neuerliche Überprüfung des bisherigen Blutdruckverlaufes mit den gespeicherten Blutdruckverläufen auf Ähnlichkeit gemäß Schritt D durchführt

## Claims

1. A therapy device for a treatment with settable target parameters, such as total ultrafiltration volume, treatment duration, maximum ultrafiltration rate, comprising a blood pressure device (24) and a regulating means (23) for a time-variable regulation of the blood pressure of a patient during a therapy procedure in dependence on detected blood pressure values, the regulating means deciding after each blood pressure measurement, in dependence on the blood pressure value detected, whether the blood regulation is continued with hypothetical or with real blood pressure values, and wherein the regulating means includes the following categories of blood pressure values in the regulation of the blood pressure:
a) blood pressure values requested by the regulating means, or substituted with values of previous therapy procedures, by regular triggers (RT) at defined times according to an implemented time schedule in dependence on the target parameters of the treatment set at the therapy device,
**characterized in that**
the regulating means (23) also includes the following categories of blood pressure values in the regulation of blood pressure:
b) blood pressure values requested by measurement and evaluation devices for other physiological parameters at other times than under a) by irregular triggers (IRT) and/or quasi-regular triggers (QRT), where irregular triggers are triggers that occur at greater time intervals from the time of the next regular trigger, whereas quasi-regular triggers are triggers that occur at shorter time intervals from the time of the next regular trigger, and
c) blood pressure values requested manually by medical staff at other times than under a) and b) by irregular triggers (IRT) and/or quasi-irregular triggers (QRT).

2. The therapy device of claim 1, wherein the regulating means (23) performs the following steps:
A acquiring, classifying and processing all blood pressure values obtained according to the categories a) to c), in dependence on the respective times of the measurements and corresponding to the schedule for the blood pressure regulation predefined by the regulating means (23),
B substituting all blood pressure values of category a) with the obtained blood pressure values of categories b) and c), if these measurement values are transferred from the blood pressure meter to the regulating means within a predetermined tolerance period immediately before the times defined for the blood pressure values of category a),
C establishing and storing an archive of the time profiles of the blood pressure in case of a plurality of therapy procedures,
D obtaining as a template (35) the blood pressure profile among the stored profiles that is most similar to the current blood pressure profile (33),
E regulating the blood pressure using the blood pressure values of categories a) to c), the values for scheduled times without blood pressure measurement being taken from the template (35).

3. The therapy device of claim 2, wherein after each blood pressure measurement the regulating means checks the current blood pressure profile again for similarity to the stored blood pressure profiles, in accordance with step D.

## Revendications

1. Dispositif de thérapie pour un traitement avec des paramètres fixés, comme le volume total de l'ultrafiltration, la durée de traitement, le taux maximal de l'ultrafiltration, comprenant un oscillomètre (24) et un moyen de régulation (23) pour réguler, de manière flexible en temps et en fonction de données de la pression artérielle déterminées, la pression artérielle d'un sujet pendant un processus de thérapie, le moyen de régulation décidant, après chaque mesurage de la pression artérielle, en fonction de la valeur de la pression artérielle déterminée, si la régulation de la pression artérielle sera continue avec des valeurs de pression artérielle hypothétiques ou réelles, et le moyen de régulation de la pression artérielle (23) incluant la catégorie suivante de la valeur de pression artérielle dans la régulation de la pression artérielle:
a) valeurs de pression artérielle, qui sont demandées par le moyen de régulation par des déclencheurs réguliers (RT) ou substituées par des valeurs de processus de thérapie antérieurs, suivant un régime chronologique implémenté et en fonction des paramètres fixés, ajustés au moyen de thérapie, à des moments prévus,
**caractérisé en ce que**
le moyen de régulation (23) aussi inclut les catégories suivantes de valeurs de pression artérielle dans la régulation de la pression artérielle:
b) valeurs de pression artérielle demandées par des appareils de mesurage et d'évaluation d'autres paramètres physiologiques à des moments autres que mentionnés dans a) par des déclencheurs irréguliers (IRT) et/ou des déclencheurs quasi-réguliers (QRT), les déclencheurs irréguliers étant des déclencheurs apparaissant dans un intervalle plus grand du moment du prochain déclencheur régulier et les déclencheurs quasi-irréguliers étant des déclencheurs apparaissant dans un intervalle plus court du moment du prochain déclencheur régulier, et
c) valeurs de pression artérielle demandées par le personnel médical à des moments autres que mentionnés dans a) par des déclencheurs irréguliers (IRT) et/ou des déclencheurs quasi-régulier (QRT).

2. Dispositif de thérapie selon la revendication 1, dans lequel le moyen de régulation (23) exécute les étapes suivantes:
A détection, classification et traitement de toutes valeurs de pression artérielle déterminées selon les catégories a) à c), en fonction des moments de mesurage respectifs et suivant le régime chronologique prédéterminé par le moyen de régulation (23) pou la régulation de la pression artérielle,
B substitution de toutes valeurs de pression artérielle selon la catégorie a) par des valeurs de pression artérielle mesurées selon les catégories b) et c), si ces valeurs de mesurage sont transférées par l'oscillomètre au moyen de régulation de la pression artérielle dans un délai de tolérance fixe immédiatement avant les moments définis pour les valeurs de pression artérielles selon la catégorie a),
C établissement et mémorisation d'une collection des développements chronologiques de la pression artérielle pour plusieurs processus de thérapie,
D détermination du développement chronologique de la pression artérielle, qui présente la plus grande similarité avec le développement actuel de la pression artérielle (33), comme directrice (35),
E régulation de la pression artérielle selon les valeurs de pression artérielles obtenues selon les catégories a) à c), les valeurs pour des moments prévus sans mesurage de la pression artérielle étant déterminés de la directrice (35).

3. Dispositif de thérapie selon la revendication 2, dans lequel, après chaque mesurage de la pression artérielle, le moyen de régulation fait une nouvelle révision du développement présent de la pression artérielle pour déterminer une similarité selon l'étape D.
